# FASCICULE DE BREVET EUROPEEN

(11) **EP 1 351 664 B1**
(45) Date de publication et mention de la délivrance du brevet: **12.08.2009**
(21) Numéro de dépôt: 02710952.9
(22) Date de dépôt: 08.01.2002
(51) Int. Cl.: A61K 8/88, A61K 8/891, A61Q 1/02, A61Q 1/10, A61Q 1/12

(54) **COMPOSITION COSMETIQUE DE TEXTURE PATEUSE A PULVERULENTE**
KOSMETIKZUSAMMENSETZUNG MIT SALBENARTIGER BIS PUDERARTIGER TEXTUR
COSMETIC COMPOSITION WITH PASTY TO POWDERY TEXTURE

(30) Priorité: 08.01.2001 FR 0100180; 03.10.2001 FR 0112733
(43) Date de publication de la demande: 15.10.2003
(73) Titulaire: L'ORÉAL, 75008 Paris (FR)
(72) Inventeur: DELACOUR, Marie-Laure, F-75013 Paris (FR); STYCZEN, Patrice, F-91190 Gif-sur-Yvette (FR); RAY, Xavier, F-91580 Villeconin (FR)
(74) Mandataire: Kromer, Christophe
(86) Numéro de dépôt international: PCT/FR2002/000044
(87) Numéro de publication internationale: WO 2002/053126

(56) Documents cités:
- EP-A- 1 023 893
- EP-A- 1 025 837
- EP-A- 1 051 968
- DATABASE CHEMICAL ABSTRACTS [en ligne] STN; access number 133; 325 487, XP002182334 & JP 2000 302638 A (KOSEI CO., LTD) 31 octobre 2000 (2000-10-31)
- DATABASE CHEMICAL ABSTRACTS [en ligne] STN; access number: 135: 126 942, XP002182335 & JP 2001 199828 A (KOSEI CO., LTD) 24 juillet 2001 (2001-07-24)

## Description

La présente invention a pour objet une composition cosmétique, notamment de maquillage ou de soin, sous forme pâteuse et/ou pulvérulente, par exemple libre, compacte, pressée, comprenant un liant particulier.

Les poudres de maquillage comprennent généralement, d'une part, une phase particulaire comportant notamment des pigments et des charges et d'autre part, une phase grasse au titre de liant comprenant des corps gras, destinée à conférer au produit fini une certaine cohésion, à donner une douceur et une propriété émolliente au produit de maquillage et à favoriser son adhérence sur la peau.

La formulation des agents liants dans les poudres, en particulier dans les poudres compactes, soulève de nombreuses difficultés car la composition finale doit être suffisamment homogène et compacte pour présenter une bonne aptitude au prélèvement et pour éviter par ailleurs une fragmentation provoquée notamment par des chocs.

Les poudres de maquillage sont donc des produits constitués généralement d'un très fort taux de composés pulvérulents secs et d'huiles. Ces produits peuvent ainsi donner des sensations de tiraillement ou un effet desséchant lorsqu'ils sont appliqués sur la peau, et n'apportent aucune sensation de fraîcheur.

D'autre part, il a été décrit dans les demandes de brevet EP-1.023.893 et EP 1.025.837 des compositions de maquillage ou de soin comprenant des particules d'un organopolysiloxane hydrophile. Dans les domaines de composition décrits, ces compositions sont des liquides qui s'écoulent et ne peuvent donc pas être mis en forme.

La Demanderesse a trouvé de manière inattendue que l'utilisation comme liant d'une suspension aqueuse d'une poudre d'un organopolysiloxane solide élastomérique au moins partiellement réticulé permettait l'obtention d'une composition à texture originale, de pâteuse à pulvérulente, qui présente d'excellentes propriétés cosmétiques en particulier en terme de fraîcheur. De plus de telles compositions peuvent avantageusement être mises en forme par pressage dans un contenant cosmétique usuel.

L'invention a donc pour objet une nouvelle composition cosmétique, notamment de maquillage ou de soin, comprenant au moins un liant A et au moins une phase particulaire B, le liant comprenant au moins une phase aqueuse et au moins un organopolysiloxane solide élastomérique au moins partiellement réticulé C, et le liant étant une suspension aqueuse dudit organopolysiloxane dans ladite phase aqueuse, ladite composition étant **caractérisée en ce qu**'elle comprend de 35 à 60 % de phase particulaire, en poids par rapport au poids total de la composition, de 25 à 45%, d'organopolysiloxane, en poids par rapport au poids total de la composition, et en ce que le rapport A/B, en poids par rapport au poids total de la composition, entre le liant A et ladite phase particulaire B, est de 1 à 1,6, et le rapport pondéral organopolysiloxane C/ phase particulaire B est de 0,6 à 1,2.

Lorsque c'est une composition de soin, la composition selon l'invention est avantageusement une composition de soin modifiant l'aspect de la surface de la peau, notamment par effets optiques. Par exemple c'est une composition matifiante et/ou masquant les imperfections de le peau telle ques que rougeurs, ridules, et/ou pores. On parle alors de composition de soin à effet matifiant.

La composition selon l'invention a une texture de poudreuse à pâteuse, originale, et présente une dureté Shore de 0 à 80, de préférence de 0 à 40, de façon encore plus préférée de 0 à 10. Les textures de type pâteuses ont généralement une dureté Shore de 0 à 10, de préférence de 0 à 5, de façon encore plus préférée aux environs de 0, par exmple de 0 à 2. Les textures de type poudreuses ont généralement une dureté Shore plus élevée, jusqu'à environ 70. Ainsi la composition selon l'invention présente une bonne cohésion et peut être utilisée facilement pour se maquiller.

Le protocole de mesure de la dureté Shore est le suivant. La dureté est mesurée sur le produit pressé généralement en coupelle. La coupelle est le plus souvent une coupelle métallique ronde de environ 50 mm de diamètre et de environ 4 mm de hauteur, telle que la coupelle de référence commerciale A96G de MEPCO. La masse de produit dans la coupelle est de 8 g environ +/- 1 g. La pression de compactage est généralement de 100 bars (où 1 bar = 0,1 MPa). La compacteuse est une compacteuse manuelle de type la compacteuse de référence commerciale Kemwall Engineering. La mesure est réalisée sur un duromètre de type ZWICK. Elle consiste à abaisser manuellement un levier pour faire descendre un mobile relié à un système de mesure de force. Lorsque toute la surface du mobile est au contact de l'échantillon, on lit la valeur de la dureté sur le cadran à aiguille. Cette mesure est exprimée en shore. Le mobile est une aiguille métallique de 1 mm de diamètre.

La composition obtenue est très homogène et le reste même après application sur la peau, et ce pendant plusieurs heures.

La composition selon l'invention présente d'excellentes propriétés cosmétiques : elle adhère suffisamment à la peau mais pas trop, elle a un toucher particulièrement doux, et elle s'applique facilement. De plus, une telle composition est très facilement redispersable dans l'eau, ce qui offre à la personne utilisatrice la possibilité de gérer elle-même la consistance et la couvrance de son maquillage selon les besoins et/ou les circonstances. D'autre part, la composition selon l'invention permet d'obtenir, après application sur la peau et/ou les muqueuses, un rendu maquillage très naturel.

De plus, la texture particulière de la composition selon l'invention permet de l'appliquer en voie sèche ou bien humide, en particulier à l'éponge et donne une grande souplesse dans l'estompage lors du dépôt à l'éponge humide.

Enfin, la composition est susceptible d'être mise en forme par pressage, typiquement à une pression de 0 (exclu) à environ 50 bars, soit de 0 (exclu) à environ 5.10⁶ Pa dans un contenant cosmétique tel que godet ou coupelle voir même un pot. L'homme du métier est apte à choisir la pression de pressage de telle sorte qu'elle permette la mise en forme (la composition doit épouser les formes du contenant cosmétique) pratiquement sans exprimer le liquide contenu dans la composition, i.e. pratiquement sans exsudation. Selon un autre test qui peut caractériser la texture originale de la composition selon l'invention, une telle composition est suffisament plastique et déformable pour que sa forme puisse être modifiée, par exemple manuellement (comme une pâte à modeler), mais suffisament solide pour être laissée, sous une forme cylindrique approximativement de 3 cm³, sur un plan horizontal à pression atmosphérique ambiante (environ 1, 013 10⁵ Pa) et à température ambiante (environ 20°C) sans changer de forme pendant une heure.

Ainsi de telles compositions peuvent avantageusement être mises en forme par pressage dans un contenant cosmétique usuel. La mise en forme par pressage génère une surface de contact entre le produit et la consommatrice (exemple surface supérieure d'une coupelle, d'un pot, voir même d'un stick) valorisant la texture originale du produit, favorisant un contact avec le produit et mettant en évidence son toucher agréable (souple et doux), donnant un aspect ludique à l'application : prélèvement au doigt ou à l'éponge ou au pinceau (petit ou gros).

La présente invention a encore pour objet un procédé de maquillage et/ou de soin, comprenant l'application au moins partielle de la composition selon l'invention, sur toute zone cutanée du corps et/ou du visage, c'est-à-dire sur la peau, les cils et sourcils, les muqueuses (intérieurs des paupières inférieures) et les semi-muqueuses (lèvres), et toute autre zone cutanée du corps et du visage. Avantageusement ; la composition selon l'invention se présente donc sous forme d'une composition de teint, de fard à joues, de fard à sourcils, de fards à paupières, de produit anti-cernes, de produit de soin à effet matifiant, ou de produit de maquillage du corps, destinée à être appliquée au moins partiellement sur le visage et/ou sur le corps.

La composition cosmétique selon l'invention comprend de 35 à 60 % de phase particulaire, en poids par rapport au poids total de la composition. Par exemple une valeur comprise de 40 à 50% de phase particulaire, en poids par rapport au poids total de la composition, donne de bons résultats selon l'invention.

La composition cosmétique selon l'invention comprend de 25 à 45%, d'organopolysiloxane, en poids de polymère par rapport au poids total de la composition. L'organopolysiloxane joue en fait le rôle de coussin interparticulaire souple.

D'autres caractéristiques, aspects et avantages de la présente invention apparaîtront à la lecture de la description détaillée qui va suivre.

Par « élastomérique » on entend un matériau souple, déformable ayant des propriétés viscoélastiques et notamment la consistance d'une éponge ou d'une sphère souple. Son module d'élasticité est tel que ce matériau résiste à la déformation et possède une capacité limitée à l'extension et à la contraction. Ce matériau est capable de retrouver sa forme originelle suite à un étirement. Cet élastomère est formé de chaînes polymériques de haut poids moléculaire dont la mobilité est limitée par un réseau uniforme de points de réticulation.

Les organopolysiloxanes élastomériques de la composition de l'invention ne sont pas desséchants pour la peau et apportent de bonnes propriétés cosmétiques, notamment de douceur et de matité. Ces nouveaux élastomères conduisent à des compositions confortables à l'application, de bon étalement, douces et non collantes au toucher. Ces composés confèrent une bonne rémanence à l'eau, à l'application sur la peau et/ou les muqueuses.

La composition selon l'invention présente, en plus, des avantages ci-dessus, une bonne stabilité.

Les organopolysiloxanes élastomériques conformes à l'invention sont des composés partiellement ou totalement réticulés et de structure tridimensionnelle.

Les élastomères compris dans la composition selon l'invention se présentent sous forme de dispersion aqueuse de poudre contenant un organopolysiloxane élastomère solide de structure tridimensionnelle, dispersé dans de l'eau. La dispersion (ou suspension) des particules est sensiblement homogène.

Les organopolysiloxanes élastomériques selon l'invention peuvent être choisis parmi les polymères réticulés décrits dans la demande JP-A-10/175816.

Les particules d'organopolysiloxanes sont sous la forme de particules solides déformables ayant une certaine dureté, mesurable avec un duromètre Shore A (selon la norme ASTM D2240) à la température ambiante ou avec la méthode japonaise JIS-A. Cette dureté JIS peut être mesurée sur un bloc d'élastomère préparé à cet effet comme suit : mélange de l'organopolysiloxane (i) et de l'organosiloxane (ii) ; élimination de l'air du mélange ; moulage et vulcanisation au four à 100°C pendant 30 minutes ; refroidissement à température ambiante puis mesure de la dureté. La densité est aussi déterminée sur ce bloc d'élastomère.

En particulier, la dureté JIS est inférieure ou égale à 80 , de préférence inférieure ou égale à 65. Les organopolysiloxanes de la composition de l'invention sont par exemple ceux commercialisés sous les noms BY 29-122, BY 29-119, HMW 2220 et DC9509, par la société Dow-Corning Toray Silicone. On peut aussi utiliser un mélange de ces produits commerciaux. Un bloc d'élastomères selon le produit BY-29122 présente une dureté de 7 et selon le produit BY-29119 une dureté de 30. La densité est de 0,97 à 0,98.

En particulier, les particules d'organopolysiloxane élastomérique (en matière active) ont une taille comprise entre 0,1 à 500 µm, de préférence comprise entre 0,1 et 200 µm. Ces particules peuvent être sphériques, plates ou amorphes avec, de préférence, une forme sphérique.

La composition selon l'invention peut contenir en outre une phase grasse, comprenant au moins un corps gras choisi parmi les corps gras liquides à température ambiante, appelées huiles, tels que ceux décrits dans le document JP-A-10175816, les cires et les gommes, généralement solides à température ambiante, les corps gras pâteux, d'origine animale, végétale, minérale ou synthétique, et leurs mélanges. Parmi les huiles, se trouvent les huiles siliconées, fluorées, fluorosiliconées, hydrocarbonées éventuellement partiellement silicosées. Ces huiles peuvent être volatiles à température ambiante et pression atmosphérique. Par huile volatile, on entend en particulier une huile susceptible de s'évaporer, en moins d'une heure, au contact de la peau ou des lèvres. Ces huiles peuvent représenter de 0 à 30 %, de préférence de 0,1 à 15 %, en poids par rapport au poids total de la composition.

Comme huiles utilisables dans la composition de l'invention, on peut citer notamment:
- les huiles hydrocarbonées d'origine animale telles que le perhydrosqualène ;
- les huiles hydrocarbonées végétales telles que les triglycérides liquides d'acides gras, par exemple les huiles de tournesol, de maïs, de soja, de courge, de pépins de raisin, de sésame, de noisette, d'abricot, de macadamia, de ricin, d'avocat, les triglycérides des acides caprylique/caprique comme ceux vendus par la société Stearineries Dubois ou ceux vendus sous les dénominations Miglyol 810, 812 et 818 par la société Dynamit Nobel ;
- les huiles de formule R¹COOR² dans laquelle R¹ représente le reste d'un acide gras supérieur comportant de 7 à 19 atomes de carbone et R² représente une chaîne hydrocarbonée ramifiée contenant de 3 à 20 atomes de carbone comme par exemple l'huile de Purcellin ;
- les hydrocarbures linéaires ou ramifiés, d'origine minérale ou synthétique tels que les huiles de paraffine volatiles ou non et leurs dérivés, la vaseline, les polydécènes, le polyisobutène hydrogéné tel que le parléam ;
- les esters et les éthers de synthèse comme le myristate d'isopropyle, des octanoates, décanoates ou ricinoléates d'alcools ou de polyalcools ;
- des alcools gras comme l'octyl dodécanol ou l'alcool oléique ;
- les huiles fluorées partiellement hydrocarbonées et/ou siliconées comme celles décrites dans le document JP-A-2-295912 ;
- les huiles siliconées comme les polyméthylsiloxanes à chaîne siliconée linéaire ou acyclique, liquides ou pâteux à température ambiante, les phényl diméthicones, les phényl triméthicones et les polyméthylphénylsiloxanes ; et
- leurs mélanges.

Dans un mode de réalisation préféré de l'invention, la composition selon l'invention comprend des huiles siliconées. De préférence de telles huiles siliconées sont amphiphiles, type dimethicone copolyol.

Avantageusement, la composition selon l'invention peut contenir des cires hydrocarbonées, fluorées ou siliconées ou leurs mélanges, qui peuvent être solides ou semi-solides (sous forme d'une pâte) à température ambiante. Ces cires peuvent être d'origine végétale, minérale, animale et/ou synthétique. En particulier, ces cires présentent une température de fusion supérieure à 25 °C et mieux supérieure à 45 °C.

Les cires siliconées peuvent être des cires comportant une structure siliconée et des motifs à une ou plusieurs chaînes alkyle ou alcoxy pendantes et/ou en bout de structure siliconée, ces chaînes étant linéaires ou ramifiées et comportant de 10 à 45 atomes de carbone. Ces cires sont appelées respectivement des alkyldiméthicones et des alcoxydiméthicones. Par ailleurs, ces chaînes alkyle peuvent comporter une ou plusieurs fonctions ester.

Comme autres cires utilisables dans l'invention, on peut citer les cires d'origine animale comme la lanoline, la cire d'abeille ; les cires d'origine végétale telles que la cire de Carnauba ou de Candellila ; les cires d'origine minérale, par exemple de paraffine, de lignite ou les cires microcristallines, la cérésine ou l'ozokérite ; les cires synthétiques comme les cires de polyéthylène ; leurs mélanges.

Les cires peuvent être avantageusement ajoutées sous forme de microdispersion aqueuse de cire, pour apporter par exemple de la tenue et/ou de la matité et/ou pour augmenter la cohésion du produit pressé en contenant cosmétique telle qu'une coupelle._Une telle microdispersion de cires est par exemple telle qeu décrite dans le brevet EP - B1-0.446.094 Elle peut aussi être telle que commercialisée sous les références commerciales suivantes : Polygen PE et WE 1 par BASF, Aquacer 498 à 608 par BYK CERA, telle que l' Aquacer 510 et l'Aquacer 608, ou l'Aquacer 533ou bien encore la Super Oxybrill par Tiscco, ou enfin la Microdispersion 411 par Mocropowders.

Ces corps gras peuvent être choisis de manière variée par l'homme du métier afin de préparer une composition ayant les propriétés, par exemple de consistance ou de texture, souhaitées.

D'une manière générale, la composition peut comprendre de 0 (exclu) à 30%, plus généralement de 0,1 à 30% et de préférence de 0,1 à 10% de cire, en poids par rapport au poids total de la composition.

Avantageusement, la composition de l'invention contient au moins un gélifiant de phase aqueuse, à savoir un composé capable de donner l'aspect d'un gel à la composition et de l'épaissir. Ce gélifiant peut être présent dans la composition selon les quantités habituellement utilisées et par exemple à raison de 0 (exclu) à 20%, de préférence de 0,1 à 10%, en poids par rapport au poids total de la composition. Parmi les gélifiants de phase aqueuse utilisables selon l'invention, on peut citer : les gélifiants cellulosiques hydrosolubles tels que l'hydroxyéthylcellulose, la méthylcellulose, l'hydroxypropylcellulose et la carboxyméthylcellulose ; la gomme de guar ; la gomme de guar quaternisée ; les gommes de guar non-ioniques comprenant des groupements hydroxyalkyle en C₁-C₆ ; les gommes de xanthane, de caroube, de scléroglucane, de gellane, de karoya ; les alginates, la maltodextrine, l'amidon et ses dérivés, l'acide hyaluronique et ses sels ; les argiles et notamment les montmorillonites, les hectorites ou bentones, les laponites ; les polymères à groupement carboxylique comme les acides polyacryliques réticulés au moins partiellement neutralisé tels que les «Carbopol» ou «Carbomer» de la Société Goodrich (Carbomer 980 par exemple neutralisé par de la triéthanolamine -TEA en abréviation-) ; les polymères poly(métha)crylates de glycéryle ; la polyvinylpyrrolidone ; l'alcool polyvinylique ; les polymères et les copolymères réticulés d'acrylamide ; les homopolymères réticulés de chlorure de méthacryloyloxyéthyltriméthylammonium ; les polyuréthanes associatifs, les polyamides associatifs et leurs mélanges.

Selon l'invention, le gélifiant de phase aqueuse est de préférence choisi parmi la gomme de xanthane, les argiles, les polyuréthanes associatifs, les polyamides associatifs, les épaississants cellulosiques, notamment l'hydroxyéthyl cellulose, les acides polyacryliques réticulés au moins partiellement neutralisés, et leurs mélanges.

La composition selon l'invention peut aussi comprendre au moins un produit susceptible de limiter au moins en partie l'évaporation de l'eau, par exemple pratiquement totalement. Un tel produit peut par exemple se lier à l'eau de façon à empêcher au moins en partie son évaporation. Une composition dans laquelle un tel produit a été incorporé présente généralement au moins l'une des deux propriétés suivantes, selon les protocoles de mesure explicités ci-après : une absence de production de gouttes à l'intérieur d'un pot et/ou une diminution de l'activité en eau de la composition.

Pour mesurer ces propriétés, on fabrique la composition comprenant 5% dudit produit. Deux tests sont appliqués, nommées respectivement test de goutte et test d'activité en eau.

Pour le 1^{er} test, dit test de goutte, on conditionne juste après leur fabrication 300 g de composition à tester dans un pot en verre de contenance 750 ml, de diamètre externe 98 mm +/- 1 mm et de hauteur 142 mm +/- 1 mm, du type pot en verre de référence commerciale 'Bocal Twist Off 750 ml, H02768' de la Société Parisienne de Verrerie. Ce pot est fermé par un couvercle en métal de 82 mm de diamètre interne qui vient se visser dessus, de type 'Couvercle en métal blanc 82 mm, R27182', de la Société Parisienne de Verrerie). On s'assure de la bonne fermeture du bocal par le couvercle en le serrant suffisamment, et de préférence on s'assure de l'étanchéité en entourant le couvercle + au minimum 3 cm du haut du bocal avec un film plastique étirable de laboratoire de type Parafilm® de la société American National Can. L'ensemble 'formule dans bocal fermé par couvercle + Parafilm®' est placé à température ambiante (20-25°C), à pression atmosphérique (1, 013. 10⁵ Pa). On observe les parois internes du bocal toutes les 24h entre t = 0 et t = 10 jours. Si aucune gouttelette d'eau n'est perceptible à l'oeil nu sur les parois pendant cette période d'observation, le produit est un produit utilisable dans la composition selon l'invention.

Pour le second test, dit test d'activité en eau, on mesure l'activité de l'eau sur un appareil de type ROTRONIC. Cet appareil ROTRONIC est constitué de trois organes principaux : un bain-marie, un capteur de mesure, un système de lecture de la mesure. Les valeurs données ont été obtenues en utilisant les organes des types suivants : bain-marie thermostaté Ecoline RE 204 LAUDA, Hygromètre AwV C ROTRONIC (capteur de mesure) relié au bain-marie thermostaté, et Hygroscope BT-RS1 ROTRONIC (lecture de la mesure d'activité en eau) relié à l'hygromètre. L'échantillon (environ 3g) est placé dans une cuvette échantillon à usage unique type PS14 (diamètre 46mm x 16mm). L'ensemble échantillon et cuvette est placé dans la chambre de mesure qui est thermostatée à 23°C. On laisse ainsi l'ensemble se stabilisé en températrue pendant 30 minutesaprès lesquelle on lit la valeur de l'activité en eau ou Aw.. Si la valeur de l'Aw est inférieure ou égale à 0.95 +/- 0.005, le produit est un produit utilisable dans la composition selon l'invention.

Un tel produit est par exemple choisi dans le groupe formé par les sucres tel que le tréhalose, les polyols et les glycols tels que la glycérine, le polyéthylène glycol et le propylène glycol. De préférence un tel produit est la glycérine. Un tel produit est présent dans la composition selon l'invention à une teneur de 0 (exclu) à 30%, plus généralement de 0,1 à 30%, de préférence de 0,1 à 10%, en poids par rapport au poids de la composition totale.

La composition selon l'invention peut se présenter sous la forme d'une pâte ou d'une poudre, par exemple compactée, libre, ou pressée.

La composition selon l'invention peut se présenter sous forme d'une composition de de teint, de fard à joues, de fards à paupières, de fards à sourcils, d'un produit anti cernes, d'un produit matifiant, destinée à être appliquée au moins partiellement sur le visage ou sur le corps.

Bien entendu la composition de l'invention doit être cosmétiquement ou dermatologiquement acceptable, à savoir non toxique et susceptible d'être appliquée sur la peau (y compris l'intérieur des paupières) ou les lèvres d'êtres humains.

Les compositions selon l'invention comprennent également une phase particulaire qui peut comprendre au moins un pigment et/ou au moins une nacre et/ou au moins une charge et/ou des paillettes, habituellement utilisés dans les compositions cosmétiques, et/ou leurs mélanges, servant à colorer et/ou opacifier et/ou donner du corps à ladite composition selon l'invention de façon à favoriser son utilisation en maquillage et/ou en soin. Par phase particulaire on entend une phase comprenant des composants sous forme de particules, qui peuvent être de différentes tailles et/ou de différentes natures, et constituée pratiquement essentiellement de telles particules. Ces particules peuvent être sphériques, plates, en aiguilles ou sans forme différentiée.

Les particules de la phase particulaire peuvent notamment être enrobées par au moins un composé siliconé tels que des polydiméthylsiloxanes et/ou par des polymères, notamment des polyéthylènes ou des polyméthacrylates et/ou au mins un composé fluoré et/ou au moins un amino acide et/ou un enrobage minéral sous forme de couche continue ou discontinue ou de particules comme par exemple de silice.

Par pigment, il faut comprendre au moins une particule blanche ou colorée, minérale ou organique, insoluble dans le milieu, destinée à colorer et/ou opacifier la composition.

Le pigment peut être présent à une teneur allant de 0 (exclu) à 60%, plus généralement de 0,05 à 60 % en poids, de préférence allant de 0,5 à 50 %, par rapport au poids total de la composition. Il peut être de taille usuelle ou nanométrique. Le pigment est généralement choisi parmi les dioxydes de titane, de zirconium et de cérium, les oxydes de zinc, de fer et de chrome, les nanotitanes, les nanozincs, le bleu ferrique et leurs mélanges. Parmi les pigments organiques, on peut citer le noir de carbone, les laques comme les sels de calcium, de baryum, d'aluminium et de zirconium, de colorants acides tels que les colorants halogéno-acides, azoïques et anthraquinoniques et leurs mélanges. On peut aussi envisager de mélanger au moins un pigment minéral et au moins un pigment organique.

Le pigment peut notamment être enrobé par au moins un composé siliconé tels que des polydiméthylsiloxanes et/ou par des polymères, notamment des polyéthylènes, ou bien par un composé minéral par exemple sous forme de couche continue ou discontinue ou de particules comme par exemple de silice. On peut ainsi citer les « oxydes SI » qui sont des pigments enrobés polyméthylhydrogénosiloxane vendus par la société Myioshi.

Par charges, il faut comprendre des particules incolores ou blanches, minérales ou de synthèse, destinées à donner du corps ou de la rigidité à la composition, et/ou de la douceur, et/ou de la matité et/ou de l'uniformité au maquillage, ainsi qu'à contribuer au bon délitage de la composition lors de la prise et l'application.

La charge peut être présente dans la phase particulaire, à une teneur allant de 0 exclu) à 60%, plus généralement de 0,05 à 60 % en poids, de préférence de 20% à 60%, en poids par rapport au poids total de la composition. Elle peut être minérale ou de synthèse. Elle est généralement choisie parmi le talc, notamment traité en surface pour le rendre hydrophile, le mica, la silice, le kaolin, les poudres de Nylon ® (Orgasol® notamment de chez Atochem), de polyéthylène, le Téflon®, , les séricites, les argiles, l'amidon, le nitrure de bore, les poudres de polymères de tétrafluoroéthylène, les poudres de polyméthylméthacrylate, les poudres de polyuréthanne tel que le BPD-500 de la société KOBO, les poudres de polystyrène, les poudres de polyester, les microsphères creuses synthétiques telles que l'Expancel (® de la Société NOBEL INDUSTRIE), les micro éponges comme le Polytrap (® de la Société DOW CORNING) et les microbilles de résine de polyméthylsilsesquioxane (Tospearl® de la Société TOSHIBA, par exemple), les oxydes de zinc et de titane, les oxydes de zirconium et de cérium, le carbonate de calcium précipité, le carbonate et l'hydrocarbonate de magnésium, l'hydroxyapatite, les microsphères de silice creuses (Silica Beads® de la Société MAPRECOS), les microcapsules de verre et de céramique, les savons métalliques dérivés d'acides organiques carboxyliques ayant de 8 à 22 atomes de carbone, de préférence de 12 à 18 atomes de carbone, par molécule, tels que le stéarate de zinc, de magnésium et de lithium, le laurate de zinc, le myristate de magnésium, et leurs mélanges. De préférence la charge est choisie parmi le mica, le talc de préférence hydrophile, les microsphères creuses synthétiques, la poudre de polyuréthanne et les poudres de Nylon ®.

Par nacres, il faut comprendre des particules irisées qui réfléchissent la lumière.

La nacre peut être présente dans la composition à une teneur allant de 0 (exclu) à 60%, plus généralement de 0,05 à 60% en poids, de préférence de 10 à 50% en poids, en poids par rapport au poids total de la composition. Parmi les nacres envisageables, on peut citer la nacre naturelle, le mica recouvert d'oxyde de titane, d'oxyde de fer, d'hydroxyde d'aluminium, d'hydroxyde de magnésium, de silice, de pigment naturel et d'oxychlorure de bismuth ainsi que le mica titane coloré, et leurs mélanges.

Les compositions selon l'invention peuvent également comprendre des paillettes.

Selon un mode de réalisation préféré de l'invention, la phase particulaire comprend au moins un composé choisi dans le groupe formé par le mica, les nacres, le talc de préférence hydrophile, les microsphères creuses synthétiques, la poudre de polyuréthanne, les pigments et les poudres de Nylon ®.

La composition selon l'invention peut aussi comprendre au moins un colorant hydrosoluble, notamment le jus de betterave et/ou le bleu de méthylène, qui peut représenter de 0 (exclu) à 6%, de préférence de 0 ,05 à 6 %, en poids par rapport au poids total de la composition.

La composition selon l'invention peut aussi comprendre au moins un conservateur couramment utilisé par l'homme du métier, tels que le phénoxyéthanol, les parabènes, la chlorophenesine, l'alcool benzylique, le gluconate de chlorhexidine, le mélange de DMDM/HMDM hydantoin + butane-1,3-diol + iodo-propynylbytylcarbamate (70/4.5/2.5) dans l'eau ( de type Glydant plus liquide de chez LONZA), un mélange alcool ethylique + penthylene glycol + sodium methyl paraben de préférence dans les proportions 47/47/6 tel que décrit dans la demande de brevet européen EP-0.935.960, un mélange penthylene glycol + sodium methyl paraben, et leurs mélanges, à une teneur comprise de 0 (exclu) à un pourcentage suffisant pour protéger la formule, ainsi qu'il est connu de l'homme du métier, généralement au maximum de 10%, en poids par rapport au poids total de la composition, par exemple à une teneur comprise de 0 (exclu) à 2%, en poids par rapport au poids total de la composition.

Selon un mode de réalisation de l'invention, la composition selon l'invention comprend, en poids par rapport au poids total de la composition :
- De 25 à 45 %, d'organopolysiloxane C,
- De 15 à 25 %, de phase aqueuse D, et ,
- De 35 à 60 %, de Phase particulaire B

Et telle que le rapport (C+D)/B, en poids par rapport au poids total de la composition, entre la somme de l'organopolysiloxane C et la phase aqueuse D, et ladite phase particulaire B, est de 0,6 à 1,6, de préférence de 1 à 1,6.

De plus, une telle composition a généralement une dureté Shore de 0 à 70, de façon préférée de 0 à 25.

Selon un mode de réalisation de l'invention, la composition selon l'invention comprend, en poids par rapport au poids total de la composition :
- De 40 à 70 %, d'un liant A constitué de 63% d'organopolysiloxane C et de 37% d'eau, et
- De 35 à 60 %, de phase particulaire B

Et telle que le rapport A/B, en poids par rapport au poids total de la composition, entre le liant A et ladite phase particulaire B, est de 1 à 1,6.

De plus, une telle composition a généralement une dureté Shore de 0 à 70, de façon préférée de 0 à 25.

La composition selon l'invention peut comprendre en outre une phase aqueuse non apportée par le liant, de teneur comprise de 0 (exclu) à 20%, de préférence de 0 (exclu) à 10% , plus généralement de 0,01 à 10%, en poids par rapport au poids de la composition totale.

Bien entendu l'homme du métier veillera à choisir les éventuels additifs complémentaires et/ou leur quantité de telle manière que les propriétés avantageuses de la composition selon l'invention ne soient pas, ou substantiellement pas, altérées par l'adjonction envisagée. En particulier, ces additifs ne devront pas nuire à l'homogénéité, à la stabilité, au confort et à la fraîcheur de la composition.

La composition selon l'invention peut être fabriquée à froid ou par chauffage d'au moins un organopolysiloxane élastomérique sous forme de poudre dispersée dans de l'eau, ajout d'une phase particulaire, par exemple d'un ou plusieurs pigments et/ou d'une ou plusieurs charges, et/ou d'au moins un autre composé, ajout éventuel d'une phase grasse à l'état liquide (notamment portée à la température de fusion des cires la plus élevée), puis homogénéisation, par exemple émulsification, si nécessaire.

Elle peut aussi être obtenue par malaxage/extrusion comme décrit dans la demande EP-A-667 146. Ce procédé consiste à malaxer la pâte (liants + additifs + charges + pigments) pendant le refroidissement à l'aide d'un broyeur à cylindres ou d'un extrudeur-mélangeur bi-vis, tel que le mélangeur extrudeur-cuiseur « BC21 » de la société « CLEXTRAL ». Ce procédé permet l'obtention d'une composition sous forme de pâte consistante déformable.

Les compositions selon l'invention avec des teneurs en phase particulaire supérieure à 85% peuvent aussi être préparées par les mélangeurs granulateurs usuels pour l'homme du métier, par exemple de marque Baker ou Lödige.

Une méthode particulière de préparation de la composition selon l'invention comprend l'utilisation d'au moins une méthode de malaxage, de préférence un malaxage par extrusion bi-vis. Un mode de réalisation particulier de cette méthode de préparation, avec utilisation d'un extrudeur bi-vis, tel que le mélangeur extrudeur-cuiseur « BC21 » de la société « CLEXTRAL », comprend l'introduction d'une phase particulaire sous forme pulvérulente en.tête d'un extrudeur bi-vis par au moins un moyen d'introduction de ladite phase, par exemple un doseur de type doseur pondéral, l'introduction d'une suspension aqueuse d'organopolysiloxane par au moins un moyen d'introduction de ladite suspension, par exemple une pompe de type pompe péristaltique, puis un malaxage à froid dans l'extrudeur. Le boudin souple ainsi obtenu en sortie d'extrudeur est ensuite prélevé, mis en forme par exemple en coupelle puis pressé, à pression suffisante pour permettre cette mise en forme sans exprimer le liquide contenu dans le matériau formant ledit boudin, de façon à épouser la forme de ladite coupelle.

Quel que soit leur état physique (liquide ou pulvérulent), les additifs (tout ou partie), notamment le conservateur, peuvent généralement être introduits dans l'extrudeur de trois façons différentes. Une première façon consiste à utiliser une alimentation indépendante de celle de la phase particulaire et de celle de la suspernsion aqueuse : les additifs sous forme liquide sont introduits par pompe péristaltique aux fourreaux adaptés ; les additifs pulvérulents sont introduits par doseur pondéral poudre aux fourreaux adaptés. Une deuxième façon consiste à alimenter ledit additif dans la suspension aqueuse à condition de ne pas provoquer une trop forte interaction avec celle-ci, ainsi qu'il est connu de l'homme du métier. Par exemple un telle interaction peut être une prise en masse ou une augmentation de la viscosité rédhibitoire à un pompage du mélange. Enfin selon une troisième façon, dans le cas d'une interaction avec la suspension aqueuse réticulée et à condition que le taux d'additif soit inférieur à 15 %, il peut être avantageusement incorporé dans la phase pulvérulente. Le mélange de la phase pulvérulente et de l'additif, en particulier du conservateur, peut être réalisé dans un mélangeur à poudres type Baker et a généralement lieu avant l'étape d'extrusion/malaxage. Le mélange obtenu à la sortie du Baker présente un aspect de poudre libre et peut être introduit dans l'extrudeur/malaxeur via un doseur pondéral poudre de type doseur K-TRON.

La composition selon l'invention se présente dans ce cas sous la forme d'une pâte consistante à aspect poudré.

Les exemples de compositions ci-après sont donnés à titre illustratif de l'invention et sans caractère limitatif.

### EXEMPLES DE REALISATION

Les compostions selon l'invention sont réalisées, à l'exception de la troisième formule de maquillage et de la seconde formule de soin, selon le mode opératoire suivant, de fabrication sur extrudeur bi-vis corotatives, qui est un mélangeur extrudeur-cuiseur « BC21 » de la société « CLEXTRAL » :
- introduction des nacres et/ou pigments et/ou charges sous forme pulvérulente en tête d'extrudeur par un doseur pondéral,
- introduction de la suspension aqueuse de poly diméthyl siloxane réticulé (BY-29119 de la société Dow Corning) par pompe péristaltique à l'un des trois premiers fourreaux de l'extrudeur,
- introduction des autres additifs éventuels sous forme liquide aux fourreaux adaptés par exemple par pompes péristaltiques, et
- malaxage à froid dans l'extrudeur.

Le boudin souple obtenu en sortie d'extrudeur est prélevé manuellement, mis en coupelle et pressé pour épouser la forme de la coupelle. La pression utilisée doit permettre cette mise en forme sans exprimer le liquide contenu dans le matériau du boudin.

Une première formule nacrée selon l'invention a la composition suivante :

| | |
|---|---|
| Sel sodique de Ponceau | 0,06 % |
| Mica-oxyde de titane / oxyde de fer brun | 40 % |
| Jaune de Quinoléine | 0,06 % |
| BY-29119* | 59,88 % |

| | |
|---|---|
| * le BY29-119 contient 63 % de Matière active, c'est à dire 63 % de poudre d'organopolysiloxane hydrophile. | |

Le rapport pondéral liant BY-29119 / phase particulaire est de 1,49, et le rapport pondéral polymère (dans le BY-29119)/ phase particulaire est de 0,94.

On obtient ainsi un fard à paupières, de teinte brun - cuivré, très agréable au toucher et qui donne une sensation de fraîcheur une fois appliquée sur la peau des paupières.

Une deuxième formule selon l'invention a la composition suivante :

| | |
|---|---|
| Oxyde de titane (anatase non traité) | 6,74 % |
| Oxyde de fer jaune | 1,57 % |
| Oxyde de fer brun, jaune | 1,35 % |
| Oxyde de fer noir | 0,34 % |
| Poudre de Nylon (Orgasol® de chez Atochem) | 30 % |
| BY-29119 | 60 % |

Le rapport pondéral liant BY-29119 / phase particulaire est de 1,5, et le rapport pondéral polymère (dans le BY-29119)/ phase particulaire est de 0,945.

On obtient ainsi un produit de teint, très agréable au toucher et qui donne une sensation de fraîcheur une fois appliquée sur la peau du visage. Le maquillage ainsi réalisé est particulièrement remarquable par ses aspects confortable, doux, naturel et poudré.

Une troisième formule selon l'invention a la composition suivante :

| | |
|---|---|
| Oxyde de titane (anatase non traité) | 6,74 % |
| Oxyde de fer jaune | 1,57 % |
| Oxyde de fer brun, jaune | 1,35 % |
| Oxyde de fer noir | 0,34 % |
| Poudre de Nylon (Orgasol® de chez Atochem) | 25 % |
| Mica enrobé de silices sphérique et poreuse (50/30/20) (Velvetveil H6400 de la Société CATALYSTS & CHEMICALS) . | 5% |
| BY-29119 | 54 % |
| Glycérine | 5% |
| Conservateur Phenochem de la société Sharon Lab. | 1% |

Le rapport pondéral liant BY-29119 / phase particulaire est de 1,35, et le rapport pondéral polymère (dans le BY-29119)/ phase particulaire est de 0,85.

Elle peut être réalisée de deux façons différentes : soit par mélange glycérine - conservateur et alimentation de l'extrudeur - malaxeur via une pompe péristaltique, soit par introduction du conservateur dans la phase pulvérulente comme cela est explicité dans la description en troisième façon d'introduire l'additif, ici le conservateur.

On obtient ainsi, quel que soit le mode de réalisation parmi les deux modes de réalisation décrits ci-dessus, un produit de teint, très agréable au toucher et qui donne une sensation de fraîcheur une fois appliquée sur la peau du visage. La texture se diffère de l'exemple précédent par son toucher « crémeux » et sa grande homogénéité d'aspect, le maquillage ainsi réalisé est aussi remarquable sur les mêmes critères.

Les trois compositions de maquillage selon l'invention présentent des propriétés remarquables de souplesse de prise , de confort d'application et de fraîcheur à l'application. De plus de telles compositions permettent de gérer facilement la couvrance et l'intensité du dépôt, grâce à leur consistance ajustable par la dilution à l'utilisation, ce qui en fait des produits « sur mesure » très prisés des utilisateurs.

De même on peut fabriquer des compositions selon l'invention de soin matifiantes. De telles compositions de soin ont par exemple les compositions des exemples 2 et 3 de maquillage précédents selon l'invention, dans lesquelles on a remplacé les pigments et colorant par de la poudre de Nylon.

On obtient ainsi les compositions de soin matifiantes selon l'invention suivantes : Première formule de soin matifiant :

| | |
|---|---|
| Poudre de Nylon (Orgasol® de chez Atochem). | 40 % |
| BY-29119 | 60 % |

On obtient ainsi un produit de soin matifiant, très agréable au toucher et qui donne une sensation de fraîcheur une fois appliqué sur la peau du visage.

Le rapport pondéral liant BY-29119 / phase particulaire est de 1,50, et le rapport pondéral polymère (dans le BY-29119)/ phase particulaire est de 0,945.

Seconde formule de soin matifiant :

| | |
|---|---|
| Poudre de Nylon (Orgasol® de chez Atochem) | 35 % |
| Velvetveil H6400 | 5% |
| BY-29119 | 54 % |
| Glycérine | 5% |
| Conservateur Phenochem de la société Sharon Lab. | 1% |

Le rapport pondéral liant BY-29119 / phase particulaire est de 1,35, et le rapport pondéral polymère (dans le BY-29119)/ phase particulaire est de 0,85.

On obtient ainsi un produit de soin matifiant, très agréable au toucher et qui donne une sensation de fraîcheur une fois appliqué sur la peau du visage. Cette deuxième composition se diffère de la précédente par son aspect plus crémeux et sa plus grande homogénéité d'aspect en coupelle.

## Revendications

1. Composition cosmétique comprenant au moins un liant A et au moins une phase particulaire B, le liant comprenant au moins une phase aqueuse et au moins un organopolysiloxane solide élastomérique au moins partiellement réticulé C, et le liant étant une suspension aqueuse dudit organopolysiloxane dans ladite phase aqueuse, ladite composition étant **caractérisée en ce qu'**elle comprend de 35 % à 60 % en poids, par rapport au poids total de la composition, de phase particulaire, de 25 % à 45 % en poids, par rapport au poids total de la composition, d'organopolysiloxane solide élastomérique partiellement ou totalement réticulé, le rapport pondéral liant A/ phase particulaire B étant de 1 à 1,6, et le rapport pondéral organopolysiloxane C / phase particulaire B est de 0,6 à 1,2.

2. Composition selon la revendication précédente, **caractérisée par le fait que** les particules d'organopolysiloxane ont une taille comprise entre 0,1 à 500 µm, de préférence comprise entre 0,1 et 200 µm.

3. Composition selon l'une quelconque des revendications précédentes, **caractérisée par le fait que** les particules d'organopolysiloxane présentent une dureté JIS inférieure ou égale à 80, de préférence inférieure ou égale à 65.

4. Composition selon l'une quelconque des revendications précédentes, **caractérisée par le fait que** la phase particulaire comprend des particules choisies parmi les pigments, les nacres, les charges, les paillettes, et leurs mélanges.

5. Composition selon l'une des revendications précédentes, **caractérisée par le fait que** la phase particulaire comprend des pigments.

6. Composition selon la revendication 4 ou 5, **caractérisée par le fait que** le pigment est choisi parmi les dioxydes de titane, les dioxydes de zirconium, les dioxydes de cérium, les oxydes de zinc, les oxydes de fer, les oxydes de chrome, les nanotitanes, les nanozincs, le bleu ferrique, le noir de carbone, les laques, et leurs mélanges.

7. Composition selon l'une quelconque des revendications 4 à 6, **caractérisée par le fait que** les pigments sont présents en une teneur allant de 0,05 % à 60 % en poids, par rapport au poids total de la composition, de préférence allant de 0,5 % à 50 % en poids.

8. Composition selon l'une quelconque des revendications précédentes, **caractérisée par le fait que** la phase particulaire comprend une nacre.

9. Composition selon l'une des revendications 4 ou 8, **caractérisée par le fait que** la nacre est choisie parmi la nacre naturelle, le mica recouvert d'oxyde de titane, d'oxyde de fer, d'hydroxyde d'aluminium, d'hydroxyde de magnésium, de silice, de pigment naturel et d'oxychlorure de bismuth, le mica titane coloré, et leurs mélanges.

10. Composition selon l'une des revendications 4, 8, 9, **caractérisée par le fait que** la nacre est présente en une teneur allant de 0,05 % à 60 % en poids, par rapport au poids total de la composition, de préférence allant de 10 % à 50 % en poids.

11. Composition selon l'une quelconque des revendications précédentes, **caractérisée par le fait que** la phase particulaire comprend une charge.

12. Composition selon la revendication 4 ou 11, **caractérisée par le fait que** la charge est choisie parmi le talc, le mica, la silice, le kaolin, les poudres de Nylon ®, la poudre de polyéthylène, les séricites, les argiles, l'amidon, le nitrure de bore, les poudres de polymères de tétrafluoroéthylène, les poudres de polyméthylméthacrylate, les poudres de polyuréthanne, les poudres de polystyrène, les poudres de polyester, les microsphères creuses synthétiques, les micro éponges et les microbilles de résine de polyméthylsilsesquioxane, les oxydes de zinc et de titane, les oxydes de zirconium et de cérium, le carbonate de calcium précipité, le carbonate et l'hydrocarbonate de magnésium, l'hydroxyapatite, les microsphères de silice creuses, les microcapsules de verre et de céramique, les savons métalliques d'acides organiques carboxylique ayant de 12 à 18 atomes de carbone, et leurs mélanges.

13. Composition selon l'une des revendications 4, 11, 12, **caractérisée par le fait que** la charge est choisie parmi le mica, le talc, les poudres de Nylon^{®}, les microsphères creuses synthétiques, les poudres de polyuréthanne.

14. Composition selon l'une quelconque des revendications 4,11 à 13, **caractérisée par le fait que** la charge est présente en une teneur allant de 0,05 % à 60 % en poids, par rapport au poids total de la composition, de préférence allant de 20 % à 60 % en poids.

15. Composition selon l'une quelconque des revendications précédentes, **caractérisé par le fait que** la phase particulaire comprend au moins un composé choisi dans le groupe formé par les nacres, les pigments, le mica, le talc, les microsphères creuses synthétiques, la poudre de polyuréthanne, et les poudres de Nylon^{®}.

16. Composition selon l'une quelconque des revendications 4 à 15, **caractérisée par le fait que** les particules sont enrobées par un composé choisi parmi les composés siliconés, un polyéthylène, un polyméthacrylate, un composé fluoré, un aminoacide, de la silice.

17. Composition selon l'une des revendications précédentes, **caractérisée par le fait qu'**elle comprend une phase grasse comprenant un corps gras choisi parmi les huiles, les cires, les gommes, les corps gras pâteux, d'origine animale, végétale, minérale ou synthétique, et leurs mélanges.

18. Composition selon l'une quelconque des revendications précédentes, **caractérisée par le fait qu'**elle comprend une phase grasse comprenant une microdispersion aqueuse de cire.

19. Composition selon l'une quelconque des revendications précédentes, **caractérisée par le fait qu'**elle comprend une cire en une teneur allant de 0,1 % à 30 % en poids, par rapport au poids total de la composition, et de préférence allant de 0,1 % à 10 % en poids.

20. Composition selon l'une quelconque des revendications précédentes, **caractérisée par le fait qu'**elle comprend une huile volatile.

21. Composition selon l'une des revendications précédentes, **caractérisée par le fait qu'**elle contient un gélifiant de phase aqueuse.

22. Composition selon la revendication précédente, **caractérisée par le fait que** le gélifiant de phase aqueuse est choisi parmi les gélifiants cellulosiques hydrosolubles ; la gomme de guar ; la gomme de guar quaternisée ; les gommes de guar non-ioniques comprenant des groupements hydroxyalkyle en C₁-C₆ ; les gommes de xanthane, de caroube, de scléroglucane, de gellane, de karoya ; les alginates, la maltodextrine, l'amidon et ses dérivés, l'acide hyaluronique et ses sels ; les argiles ; les acides polyacryliques réticulés au moins partiellement neutralisé ; les polymères poly(métha)crylates de glycéryle ; la polyvinylpyrrolidone ; l'alcool polyvinylique ; les polymères et les copolymères réticulés d'acrylamide ; les homopolymères réticulés de chlorure de méthacryloyloxyéthyltriméthylammonium ; les polyuréthanes associatifs, les polyamides associatifs, et leurs mélanges.

23. Composition selon la revendication 21 ou 22, **caractérisée par le fait que** le gélifiant de phase aqueuse est choisi parmi la gomme de xanthane, les argiles, les polyuréthanes associatifs, les polyamides associatifs, les épaississants cellulosiques, les acides polyacryliques réticulés au moins partiellement neutralisés, et leurs mélanges.

24. Composition selon la revendication 21 à 23, **caractérisée par le fait que** le gélifiant de phase aqueuse est présent en une teneur allant de 0,1 % à 20 % en poids, par rapport au poids total de la composition, et de préférence allant de 0,1 % à 10 % en poids.

25. Composition selon l'une des revendications précédentes, **caractérisée par le fait qu'**elle contient un produit susceptible de limiter au moins en partie l'évaporation de l'eau.

26. Composition selon la revendication 25, **caractérisée par le fait que** ledit produit est choisi parmi les sucres-et les polyols, et les glycols.

27. Composition selon la revendication 25 ou 26, **caractérisée par le fait que** ledit produit est choisi dans le groupe formé par la glycérine, le tréhalose, le polyéthylène glycol et le propylène glycol.

28. Composition selon l'une des revendications 25 à 26, **caractérisée par le fait que** ledit produit est présent en une teneur de 0,1 % à 30 % en poids, par rapport au poids de la composition, de préférence de 0,1 % à 10 % en poids.

29. Composition selon l'une des revendications précédentes, **caractérisée par le fait qu'**elle comprend en poids, par rapport au poids total de la composition :
• De 35 à 60 % de phase particulaire B,
• De 25 à 45 % d'organopolysiloxane C,
• De 15 à 25 % de phase aqueuse D,
le rapport pondéral (organopolysiloxane C + phase aqueuse D)/ phase particulaire B étant compris de 0,6 à 1,6, de préférence de 1 à 1,6.

30. Composition selon l'une des revendications précédentes, **caractérisée par le fait que** la composition est suffisament plastique et déformable pour que sa forme puisse être modifiée manuellement, et suffisament solide pour être laissée, sous une forme cylindrique de 3 cm³, sur un plan horizontal à pression atmosphérique ambiante et à température ambiante sans changer de forme pendant une heure.

31. Composition selon l'une des revendications précédentes, **caractérisée par le fait qu'**elle se présente sous forme d'une composition de fond de teint, de fard à joues, de fard à sourcils, de fards à paupières, de produit anti-cernes, de produit de maquillage du corps, de produit de soin à effet matifiant.

32. Procédé de fabrication d'une composition telle que définie dans l'une des revendications précédentes, **caractérisée par le fait que** la composition est obtenue par malaxage/extrusion.

33. Procédé selon la revendication 32, **caractérisé par le fait que** le malaxage est effectué à l'aide d'un extrudeur-mélangeur bi-vis.

34. Procédé selon la revendication 32 ou 33, **caractérisé par le fait que** la composition est mise en forme par pressage dans un contenant.

35. Procédé cosmétique de maquillage ou de soin de la peau, des lèvres, des cils, des sourcils comprenant l'application sur la peau, les lèvres, les cils, les sourcils, d'une composition selon l'une quelconque des revendications 1 à 31.

36. Utilisation d'une composition selon l'une quelconque des revendications 1 à 31 pour maquiller la peau avec une sensation de fraîcheur.

## Claims

1. Cosmetic composition comprising at least one binder A and at least one particulate phase B, the binder comprising at least one aqueous phase and at least one at least partially crosslinked elastomeric solid organopolysiloxane C, and the binder being an aqueous suspension of the said organopolysiloxane in the said aqueous phase, the said composition being **characterized in that** it comprises from 35 to 60% by weight, relative to the total weight of the composition, of particulate phase, from 25 to 45% by weight, relative to the total weight of the composition, of partially or completely crosslinked elastomeric solid organopolysiloxane, the binder A/particulate phase B weight ratio being from 1 to 1.6, and the organopolysiloxane C/particulate phase B weight ratio is from 0.6 to 1.2.

2. Composition according to the preceding claim, **characterized in that** the particles of organopolysiloxane have a size of between 0.1 and 500 µm, preferably between 0.1 and 200 µm.

3. Composition according to either of the preceding claims, **characterized in that** the particles of organopolysiloxane have a JIS hardness of less than or equal to 80, preferably of less than or equal to 65.

4. Composition according to any one of the preceding claims, **characterized in that** the particulate phase comprises particles chosen from pigments, pearlescent agents, fillers, glitter, and mixtures thereof.

5. Composition according to one of the preceding claims, **characterized in that** the particulate phase comprises pigments.

6. Composition according to Claim 4 or 5, **characterized in that** the pigment is chosen from titanium dioxides, zirconium dioxides, cerium dioxides, zinc oxides, iron oxides, chromium oxides, nanotitaniums, nanozincs, ferric blue, carbon black, lacquers, and mixtures thereof.

7. Composition according to any one of Claims 4 to 6, **characterized in that** the pigments are present in an amount ranging from 0.05% to 60% by weight, relative to the total weight of the composition, preferably ranging from 0.5 to 50% by weight.

8. Composition according to any one of the preceding claims, **characterized in that** the particulate phase comprises a pearlescent agent.

9. Composition according to either of Claims 4 and 8, **characterized in that** the pearlescent agent is chosen from natural pearl, mica coated with titanium oxide, with iron oxide, with aluminium hydroxide, with magnesium hydroxide, with silica, with a natural pigment and with bismuth oxychloride, coloured mica-titanium, and mixtures thereof.

10. Composition according to one of Claims 4, 8 or 9, **characterized in that** the pearlescent agent is present in an amount ranging from 0.05% to 60% by weight, relative to the total weight of the composition, preferably ranging from 10% to 50% by weight.

11. Composition according to any one of the preceding claims, **characterized in that** the particulate phase comprises a filler.

12. Composition according to Claim 4 or 11, **characterized in that** the filler is chosen from talc, mica, silica, kaolin, Nylon® powders, polyethylene powder, sericites, clays, starch, boron nitride, powders of tetrafluoroethylene polymers, powders of polymethyl methacrylate, polyurethane powders, polystyrene powders, polyester powders, synthetic hollow microspheres, microsponges and microbeads of poymethylsilsesquioxane resin, zinc and titanium oxides, zirconium and cerium oxides, precipitated calcium carbonate, magnesium carbonate and hydrocarbonate, hydroxyapatite, hollow silica microspheres, glass and ceramic microcapsules, metal soaps of organic carboxylic acids having from 12 to 18 carbon atoms, and mixtures thereof.

13. Composition according to one of Claims 4, 11 or 12, **characterized in that** the filler is chosen from mica, talc, Nylon® powders, synthetic hollow microspheres and polyurethane powders.

14. Composition according to any one of Claims 4, 11 to 13, **characterized in that** the filler is present in an amount ranging from 0.05% to 60% by weight, relative to the total weight of the composition, preferably ranging from 20% to 60% by weight.

15. Composition according to any one of the preceding claims, **characterized in that** the particulate phase comprises at least one compound chosen from the group consisting of pearlescent agents, pigments, mica, talc, synthetic hollow microspheres, polyurethane powder and Nylon® powders.

16. Composition according to any one of Claims 4 to 15, **characterized in that** the particles are coated with a compound chosen from silicone compounds, a polyethylene, a polymethacrylate, a fluorinated compound, an amino acid, and silica.

17. Composition according to one of the preceding claims, **characterized in that** it comprises a fatty phase comprising a fatty substance chosen from oils, waxes, gums, pasty fatty substances, of animal, plant, mineral or synthetic origin, and mixtures thereof.

18. Composition according to any one of the preceding claims, **characterized in that** it comprises a fatty phase comprising an aqueous microdispersion of wax.

19. Composition according to any one of the preceding claims, **characterized in that** it comprises a wax in an amount ranging from 0.1% to 30% by weight, relative to the total weight of the composition, and preferably ranging from 0.1% to 10% by weight.

20. Composition according to any one of the preceding claims, **characterized in that** it comprises a volatile oil.

21. Composition according to one of the preceding claims, **characterized in that** it contains an aqueous phase gelling agent.

22. Composition according to the preceding claim, **characterized in that** the aqueous phase gelling agent is chosen from water-soluble cellulosic gelling agents; guar gum; quaternized guar gum; nonionic guar gums comprising C₁-C₆ hydroxyalkyl groups; xanthan, carob, scleroglucan, gellan and karaya gums; alginates, maltodextrin, starch and its derivatives, hyaluronic acid and its salts; clays; at least partially neutralized crosslinked polyacrylic acids; polyglyceryl (meth)acrylate polymers; polyvinylpyrrolidone; polyvinyl alcohol; crosslinked polymers and copolymers of acrylamide; crosslinked homopolymers of methacryloyloxyethyltrimethylammonium chloride; associative polyurethanes, associative polyamides and mixtures thereof.

23. Composition according to Claim 21 or 22, **characterized in that** the aqueous phase gelling agent is chosen from xanthan gum, clays, associative polyurethanes, associative polyamides, cellulosic thickeners, at least partially neutralized crosslinked polyacrylic acids, and mixtures thereof.

24. Composition according to Claims 21 to 23, **characterized in that** the aqueous phase gelling agent is present in an amount ranging from 0.1% to 20% by weight, relative to the total weight of the composition, and preferably ranging from 0.1% to 10% by weight.

25. Composition according to one of the preceding claims, **characterized in that** it contains a product capable of at least partly limiting the evaporation of water.

26. Composition according to Claim 25, **characterized in that** the said product is chosen from sugars, polyols and glycols.

27. Composition according to Claim 25 or 26, **characterized in that** the said product is chosen from the group consisting of glycerine, trehalose, polyethylene glycol and propylene glycol.

28. Composition according to either of Claims 25 and 26, **characterized in that** the said product is present in an amount of 0.1% to 30% by weight, relative to the weight of the composition, preferably from 0.1% to 10% by weight.

29. Composition according to one of the preceding claims, **characterized in that** it comprises by weight, relative to the total weight of the composition:
• from 35 to 60% of the particulate phase B,
• from 25 to 45% of organopolysiloxane C,
• from 15 to 25% of aqueous phase D,
the (organopolysiloxane C + aqueous phase D)/ particulate phase B weight ratio being between 0.6 and 1.6, preferably 1 and 1.6.

30. Composition according to one of the preceding claims, **characterized in that** the composition is sufficiently plastic and deformable for its shape to be manually modified, and sufficiently solid to be left, in a cylindrical form of 3 cm³, on a horizontal plane at ambient atmospheric pressure and at room temperature without changing shape for one hour.

31. Composition according to one of the preceding claims, **characterized in that** it is provided in the form of a foundation, blusher, eyebrow make-up, eyeshadow, concealer product, body make-up product or matt-effect care product composition.

32. Method for manufacturing a composition as defined in one of the preceding claims, **characterized in that** the composition is obtained by blending/extrusion.

33. Method according to Claim 32, **characterized in that** the blending is carried out with the aid of a twin-screw extruder-mixer.

34. Method according to Claim 32 or 33, **characterized in that** the composition is shaped by pressing in a container.

35. Cosmetic method for applying make-up to or caring for the skin, the lips, the eyelashes, the eyebrows, comprising applying to the skin, the lips, the eyelashes or the eyebrows a composition according to any one of Claims 1 to 31.

36. Use of a composition according to any one of Claims 1 to 31 for applying make-up to the skin with a sensation of freshness.

## Patentansprüche

1. Kosmetische Zusammensetzung, die mindestens ein Bindemittel A und mindestens eine Partikelphase B enthält, wobei das Bindemittel mindestens eine wässrige Phase und mindestens ein festes elastomeres Organopolysiloxan C umfasst, das zumindest teilweise vernetzt ist, und wobei das Bindemittel eine wässrige Suspension des Organopolysiloxans in der wässrigen Phase ist, wobei die Zusammensetzung **dadurch gekennzeichnet ist, dass** sie 35 bis 60 Gew.-% Partikelphase, bezogen auf das Gesamtgewicht der Zusammensetzung, und 25 bis 45 G-ew.-% festes elastomeres Organopolysiloxan, das ganz oder teilweise vernetzt ist, bezogen auf das Gesamtgewicht der Zusammensetzung, enthält, wobei das Gewichtsverhältnis Bindemittel A/Partikelphase B im Bereich von 1 bis 1,6 liegt und wobei das Gewichtsverhältnis Organopolysiloxan C/Partikelphase B im Bereich von 0,6 bis 1,2 liegt.

2. Zusammensetzung nach dem vorhergehenden Anspruch, **dadurch gekennzeichnet, dass** die Organopolysiloxan-Partikel eine Größe im Bereich von 0,1 bis 500 µm und vorzugsweise 0,1 bis 200 µm besitzen.

3. Zusammensetzung nach einem der vorhergehenden Ansprüche, **dadurch gekennzeichnet, dass** die Organopolysiloxan-Partikel eine JIS-Härte von 80 oder darunter und vorzugsweise 65 oder darunter aufweisen.

4. Zusammensetzung nach einem der vorhergehenden Ansprüche, **dadurch gekennzeichnet, dass** die Partikelphase Partikel enthält, die unter den Pigmenten, Perlglanzpigmenten, Füllstoffen, Pailletten und deren Gemischen ausgewählt sind.

5. Zusammensetzung nach einem der vorhergehenden Ansprüche, **dadurch gekennzeichnet, dass** die Partikelphase Pigmente umfasst.

6. Zusammensetzung nach Anspruch 4 oder 5**, dadurch gekennzeichnet, dass** das Pigment unter Titandioxid, Zirconiumdioxid, Cerdioxid, Zinkoxiden, Eisenoxiden, Chromoxiden, Nanotitanverbindungen. Nanozinkverbindungen, Eisenblau, Ruß, Lacken und deren Gemischen ausgewählt ist.

7. Zusammensetzung nach einem der Ansprüche 4 bis 6, **dadurch gekennzeichnet, dass** die Pigmente in einer Menge von 0,05 bis 60 Gew.-%, bezogen auf das Gesamtgewicht der Zusammensetzung, und vorzugsweise 0,5 bis 50 Gew.-% enthalten sind.

8. Zusammensetzung nach einem der vorhergehenden Ansprüche, **dadurch gekennzeichnet, dass** die Partikelphase ein Perlglanzpigment umfasst.

9. Zusammensetzung nach einem der Ansprüche 4 oder 8, **dadurch gekennzeichnet, dass** das Perlglanzpigment unter natürlichem Perlmut, mit Titanoxid, Eisenoxid, Aluminiumhydroxid, Magnesiumhydroxid, Siliciumdioxid, natürlichem Pigment oder Bismutoxidchlorid beschichteten Glimmerpigmenten, farbigen Titan-Glimmerpigmenten und deren Gemischen ausgewählt ist.

10. Zusammensetzung nach einem der Ansprüche 4, 8, 9, **dadurch gekennzeichnet, dass** das Perlglanzpigment in einer Menge von 0,05 bis 60 Gew.-%, bezogen auf das Gesamtgewicht der Zusammensetzung, und vorzugsweise 10 bis 50 Gew.-% enthalten ist.

11. Zusammensetzung nach einem der vorhergehenden Ansprüche, **dadurch gekennzeichnet, dass** die Partikelphase einen Füllstoff enthält.

12. Zusammensetzung nach Anspruch 4 oder 11, **dadurch gekennzeichnet, dass** der Füllstoff unter Talk, Glimmer, Kieselsäure, Kaolin, Nylon^{®}-Pulvern, Sericiten, Tonen, Stärke, Bornitrid, Pulvern von Tetrafluorethylenpolymeren, Polymethylmethacrylatpulvern, Polyurethanpulvern, Polystyrolpulvern, Polyesterpulvern, synthetischen Mikrohohlkugeln, Mikroschwämmen und Mikrokugeln aus Polymethylsilsesquioxanharz, Oxiden von Zink und Titan, Oxiden von Zirconium und Cer, gefälltem Calciumcarbonat, Magnesiumcarbonat, Magnesiumhydrogencarbonat, Siliciumoxidmikrohohlkugeln, Mikrokapseln aus Glas und Keramik, von organischen Carbonsäuren mit 12 bis 18 Kohlenstoffatomen abgeleiteten Metallseifen und deren Gemischen ausgewählt ist.

13. Zusammensetzung nach einem der Ansprüche 4, 11, 12, **dadurch gekennzeichnet, dass** der Füllstoff unter Glimmer, Talk, Nylon^{®}-Pulvern, synthetischen Mikrohohlkugeln und Polyurethanpulvern ausgewählt ist.

14. Zusammensetzung nach einem der Ansprüche 4, 11 bis 13, **dadurch gekennzeichnet, dass** der Füllstoff in einer Menge von 0,05 bis 60 Ges.-%, bezogen auf das Gesamtgewicht der Zusammensetzung, und vorzugsweise 20 bis 60 Gew.-% enthalten ist.

15. Zusammensetzung nach einem der vorhergehenden Ansprüche, **dadurch gekennzeichnet, dass** die Partikelphase mindestens eine Verbindung umfasst, die unter den Perlglanzstoffen, Pigmenten, Glimmer, Talk, synthetischen Mikrohohlkugeln, Polyurethanpulvern und Nylon^{®}-Pulvern ausgewählt ist.

16. Zusammensetzung nach einem der Ansprüche 4 bis 15**, dadurch gekennzeichnet, dass** die Partikel mit einer Verbindung umhüllt sind, die unter den Siliconverbindungen, Polyethylenen, Polymethacrylaten, fluorierten Verbindungen, Aminosäuren und Kieselsäure ausgewählt ist.

17. Zusammensetzung nach einem der vorhergehenden Ansprüche, **dadurch gekennzeichnet, dass** sie eine Fettphase aufweist, die eine Fettsubstanz enthält, die unter den Ölen, Wachsen, Gummis, pastösen Fettsubstanzen tierischer, pflanzlicher, mineralischer oder synthetischer Herkunft und deren Gemischen ausgewählt ist.

18. Zusammensetzung nach einem der vorhergehenden Ansprüche, **dadurch gekennzeichnet, dass** sie eine Fettphase aufweist, die eine wässrige Wachsmikrodispersion enthält.

19. Zusammensetzung nach einem der vorhergehenden Ansprüche, **dadurch gekennzeichnet, dass** sie ein Wachs in einer Menge von 0,1 bis 30 Gew.-%, bezogen auf das Gesamtgewicht der Zusammensetzung, und vorzugsweise 0,1 bis 10 Gew.-% enthält.

20. Zusammensetzung nach einem der vorhergehenden Ansprüche, **dadurch gekennzeichnet, dass** sie ein flüchtiges Öl enthält.

21. Zusammensetzung nach einem der vorhergehenden Ansprüche, **dadurch gekennzeichnet, dass** sie einen Gelbildner für die wässrige Phase enthält.

22. Zusammensetzung nach dem vorhergehenden Anspruch, **dadurch gekennzeichnet, dass** der Gelbildner für die wässrige Phase unter den gelbildenden wasserlöslichen Cellulosen; Guargummi; quaternisiertem Guargummi; nichtionischen Guargummen, die C₁₋₆-Hydroxyalkylgruppen aufweisen; Xanthangummi, Johannisbrot-Kernmehl, Skleroglucan, Gellan, Karaya-Gummi; Alginaten, Maltodextrin, Stärke und ihren Derivaten, Hyaluronsäure und ihren Salzen; Tonen; vernetzten Polyacrylsäuren, die zumindest zum Teil neutralisiert sind; Polyglyceryl(meth)acrylatpolymeren; Polyvinylpyrrolidon; Polyvinylalkohol; vernetzen Polymeren und Copolymeren von Acrylamid; vernetzen Homopolymeren von Methacryloyloxyethyltrimethylammoniumchlorid; assoziativen Polyurethanen; assoziativen Polyamiden und deren Gemischen ausgewählt ist.

23. Zusammensetzung nach Anspruch 21 oder 22, **dadurch gekennzeichnet, dass** der Gelbildner für die wässrige Phase unter Xanthangummi, Tonen, assoziativen Polyurethanen, assoziativen Polyamiden, verdickenden Cellulosen, vernetzten Polyacrylsäuren, die zumindest zum Teil neutralisiert sind, und deren Gemischen ausgewählt ist.

24. Zusammensetzung nach Anspruch 21 bis 23, **dadurch gekennzeichnet, dass** der Gelbildner für die wässrige Phase in einer Menge von 0,1 bis 20 Gew.-%, bezogen auf das Gesamtgewicht der Zusammensetzung, und vorzugsweise 0,1 bis 10 Gew.-% enthalten ist.

25. Zusammensetzung nach einem der vorhergehenden Ansprüche, **dadurch gekennzeichnet, dass** sie ein Produkt enthält, das befähigt ist, zumindest teilweise die Verdampfung von Wasser zu begrenzen.

26. Zusammensetzung nach Anspruch 25, **dadurch gekennzeichnet, dass** das Produkt unter den Zuckern, Polyolen und Glycolen ausgewählt ist.

27. Zusammensetzung nach Anspruch 25 oder 26, **dadurch gekennzeichnet, dass** das Produkt unter Glycerin, Trehalose, Polyethylenglycol und Propylenglycol ausgewählt ist.

28. Zusammensetzung nach einem der Ansprüche 25 bis 26, **dadurch gekennzeichnet, dass** das Produkt in einer Mnege von 0,1 bis 30 Gew.-%, bezogen auf das Gesamtgewicht der Zusammensetzung, und vorzugsweise 0,1 bis 10 Gew.-% enthalten ist.

29. Zusammensetzung nach einem der vorhergehenden Ansprüche, **dadurch gekennzeichnet, dass** sie, bezogen auf das Gesamtgewicht der Zusammensetzung, enthält:
· 35 bis 60 % Partikelphase B,
· 25 bis 45 % Organopolysiloxan C, und
· 15 bis 25 % wässrige Phase D,
wobei das Gewichtsverhältnis (Organopolysiloxan C + wässrige Phase D)/Partikelphase B im Bereich von 0,6 bis 1,6 und vorzugsweise 1 bis 1,6 liegt.

30. Zusammensetzung nach einem der vorhergehenden Ansprüche, **dadurch gekennzeichnet, dass** die Zusammensetzung ausreichend plastisch und verformbar ist, dass ihre Form manuell verändert werden kann, und ausreichend fest, damit sie auf einer horizontalen Ebene unter Atmosphärendruck und Raumtemperatur in der Form eines Zylinders von 3 cm³ bleibt, ohne während einer Zeitspanne von einer Stunde die Form zu ändern.

31. Zusammensetzung nach einem der vorhergehenden Ansprüche, **dadurch gekennzeichnet, dass** sie als Make-up, Wangenrouge, Schminke für die Augenbrauen, Lidschatten, Produkt gegen Augenringe, Produkt zum Schminken des Körpers, Pflegeprodukt mit mattierender Wirkung vorliegt.

32. Verfahren zur Herstellung einer Zusammensetzung nach einem der vorhergehenden Ansprüche, **dadurch gekennzeichnet, dass** die Zusammensetzung durch Kneten/Extrudieren erhalten wird.

33. Verfahren nach Anspruch 32, **dadurch gekennzeichnet, dass** das Kneten mit Hilfe eines Zweischneckenextruders/Mischers durchgeführt wird.

34. Verfahren nach Anspruch 32 oder 33, **dadurch gekennzeichnet, dass** die Zusammensetzung durch Pressen in einem Behälter geformt wird.

35. Kosmetisches Verfahren zum Schminken oder für die Pflege der Haut, der Lippen, der Wimpern, der Augenbrauen, das umfasst, eine Zusammensetzung nach einem der Ansprüche 1 bis 31 auf die Haut, die Lippen, die Wimpern, die Augenbrauen aufzubringen.

36. Verwendung einer Zusammensetzung nach einem der Ansprüche 1 bis 31 zum Schminken der Haut mit einem Gefühl der Frische.
